(19) Europäisches Patentamt
European Patent Office
Office européen des brevets

(11) Veröffentlichungsnummer : **0 029 973**
**B1**

(12) **EUROPÄISCHE PATENTSCHRIFT**

(45) Veröffentlichungstag der Patentschrift : **13.06.84**

(51) Int. Cl.³ : **C 07 C 27/02**, C 07 C 31/20, C 07 C 69/003, C 07 C 33/26, C 07 C 35/08

(21) Anmeldenummer : **80107276.0**

(22) Anmeldetag : **21.11.80**

(54) **Verfahren zur Herstellung vicinaler Diole.**

(30) Priorität : **30.11.79 DE 2948256**

(43) Veröffentlichungstag der Anmeldung : **10.06.81 Patentblatt 81/23**

(45) Bekanntmachung des Hinweises auf die Patenterteilung : **13.06.84 Patentblatt 84/24**

(84) Benannte Vertragsstaaten : **BE DE FR GB IT NL**

(56) Entgegenhaltungen :
**DE-A- 2 425 761**
**DE-A- 2 820 521**
**US-A- 3 981 931**
**US-A- 4 035 427**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Zeidler, Ulrich, Dr.**
**Heinrich-Lersch-Strasse 19**
**D-4000 Düsseldorf 13 (DE)**

**EP 0 029 973 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, daß man vicinale Diole herstellen kann, indem man Diolester, die man beispielsweise durch Umsetzung von Olefinen mit Percarbonsäuren erhält, mit alkoholischer Alkalihydroxidlösung verseift, den Alkohol abtrennt und die carbonsauren Salze mit heißem Wasser auswäscht ; hierbei erhält man als Rückstand vicinale Diole. Diese Verfahrensweise ist aber für eine Herstellung in technischem Maßstab zu aufwendig.

Die DE-A-2 425 761 beschreibt ein kontinuierlich geführtes Verfahren zur Herstellung von Diolen aus Diolestern von niederen Carbonsäuren durch Umsetzung (oder Umesterung) mit niederen Alkanolen in Gegenwart von sauren oder alkalischen Katalysatoren, wobei die sich bildenden leicht flüchtigen Ester aus niederen Carbonsäuren und niederen Carbonsäuren und niederen Alkanolen abgetrennt, bzw. abdestilliert werden. Bei den Diolen, die in diesem Verfahren in Freiheit gesetzt werden, handelt es sich um 1,4-Butandiol, 1,2-Butandiol und 2-Methyl-1,3-propandiol. Diese Diole sind mit Essigsäure verestert. Zur Umesterung werden niedere Alkohole wie Methanol, Ethanol, Propanol oder Butanol verwendet. Das Verfahren muß in einer bestimmten Weise kontinuierlich geführt werden, damit es zu dem gewünschten Ergebnis führt. Wird das Verfahren dagegen diskontinuierlich geführt, so tritt auch bei langen Reaktionszeiten nicht der gewünschte Erfolg ein (siehe Beispiel 5).

Ein ähnliches Verfahren, das insbesondere für die Herstellung von Butandiol-1,4 und Butendiol-1,4 aus den entsprechenden Dioldiacetaten eingesetzt werden soll, wird in der DE-A-2 820 521 beschrieben. Auch in diesem Fall muß die Umsetzung zwischen Diolester und niederem Alkanol in einer bestimmten Weise kontinuierlich gestaltet werden, damit es zu dem gewünschten Ergebnis führt.

Andere Verfahren, z. B. Reduktion von Acyloinen, Hydrierung von α-Keto- und α-Hydroxycarbonsäuren, Hydrolyse von Halogenhydrinen oder Dihalogeniden erweisen sich in der Praxis ebenfalls als unbefriedigend.

Der Erfindung lag daher die Aufgabe zugrunde, ein gegenüber dem Stand der Technik verbessertes Verfahren zur Herstellung vicinaler Diole bereitzustellen, das die Nachteile der Verfahren des Standes der Technik nicht aufweist.

Gegenstand der Erfindung ist ein Verfahren zur Herstellung von Diolen aus Diolestern von niederen Carbonsäuren durch Umesterung mit niederen Alkoholen in Gegenwart von sauren oder basischen Katalysatoren, wobei die sich bildenden, leicht flüchtigen Ester aus niederen Carbonsäuren abgetrennt werden. Das Verfahren ist dadurch gekennzeichnet, daß man Ester aus vicinalen Diolen mit wenigstens 6 Kohlenstoffatomen und niederen Carbonsäuren mit niederen Alkoholen umsetzt.

Die vicinalen Diole entsprechen der Formel I

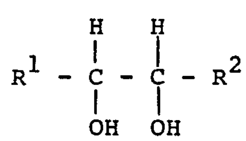

(I)

in der $R^1$ und $R^2$ Wasserstoff oder einen aliphatischen oder aromatischen Rest, der gegebenenfalls auch substituiert oder durch Heteroatome unterbrochen sein kann, mit 1-26 Kohlenstoffatomen bedeuten, mit der Maßgabe, daß $R^1$ und $R^2$ zusammen 4-28 Kohlenstoffatome aufweisen. Zu ihrer Herstellung geht man von Estern vicinaler Diole der Formel II aus.

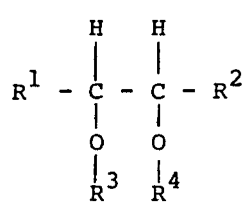

(II)

In dieser Formel haben $R^1$ und $R^2$ die oben angegebenen Bedeutungen ; von den Resten $R^3$ und $R^4$ stellt wenigstens einer einen Acylrest der Formel III

$$—OC—R^5 \qquad \text{(III)}$$

dar, in der $R^5$ Wasserstoff oder einen aliphatischen Rest mit 1-4 Kohlenstoffatomen bedeutet, während der andere Rest entweder Wasserstoff oder ebenfalls einen Alcylrest der Formel III darstellt. Diese Ester läßt man mit einem Alkanol der Formel IV

$$R^6—OH \qquad \text{(IV)}$$

in der $R^6$ einen aliphatischen Rest mit 1-4 Kohlenstoffatomen bedeutet, in Gegenwart von stark basischen oder sauren Katalysatoren unter Bildung von vicinalen Diolen der Formel I und Estern der Formel V

$$R^6—O—OC—R^5 \qquad (V)$$

in der $R^5$ und $R^6$ die für die Formeln III und IV genannten Bedeutungen haben, reagieren und destilliert den Ester der Formel V von den Diolen der Formel I ab.

Die als Ausgangsstoffe eingesetzten Ester stehen nach bekannten Verfahren, die z. B. von Malinowski, M. S., Epoxides und their Derivatives, S. 185 ff, Israel Programm for Scientific Translation, Jerusalem 1965, oder in Houben-Weyl, 1965, Bd. 6/3, S. 459 ff beschrieben sind, durch Ringspaltung end- oder innenständiger langkettiger Epoxide mit niederen aliphatischen Carbonsäuren oder aus Olefinen durch Umsetzung mit Percarbonsäuren nach J. Am. Chem. Soc. 68, 1504 (1946) zur Verfügung. Bevorzugt eingesetzte Ester leiten sich von Diolen ab, die eine lineare gesättigte Kohlenstoffkette mit 6 bis 30 Kohlenstoffatomen aufweisen und mit Ameisensäure oder Essigsäure verestert sind. Aber auch ungesättigte lineare und verzweigte, aliphatische oder aromatische Diolester können eingesetzt werden, wobei der Begriff « aliphatisch » auch aliphatisch/aromatische Kohlenwasserstoffreste umfaßt. Mono- und Diester sind dabei als reine Stoffe und im Gemisch gleichermaßen gut geeignet. Als Ausgangsstoffe geeignete Ester sind beispielsweise die vorwiegend aus dem Monoacetat oder Monoformiat bestehenden Estergemische, die man durch Umsetzung von $C_{12/14}$-Alkan-1.2-epoxid mit Essigsäure oder Ameisensäure erhält. Weiterhin geeignet sind die Umsetzungsprodukte des vicinalen n-$C_{11/14}$-Alkanoxids, dessen Oxiranring willkürlich über die Kohlenstoffkette verteilt ist, mit Essigsäure, ferner die ebenfalls vorwiegend aus dem Monoacetat bestehenden Diolester-Gemische, die sich von end- oder innenständigen vicinalen Diolen ableiten, deren Kohlenstoffkette linear oder verzweigt ist, also beispielsweise Hexandiolester-, Tetradecan-diolester- und Eicosan-diolester-Gemische. Gleichermaßen geeignet sind aber auch solche Diolester-Gemische, die zu etwa gleichen Teilen aus Dioldiestern und Diolmonoestern oder zum überwiegenden Teil aus Dioldiestern wie z. B. aus den Di-Formiaten und Di-Acetaten der zuvor genannten Diole bestehen. Auch die Mono- und Diester-Gemische von Diolen, die man durch Umsetzung von Fraktionen technischer Alkanoxid-Gemische im Kettenlängenbereich von 6 bis 30 Kohlenstoffatomen mit niederen Carbonsäuren wie Ameisensäure und Essigsäure erhält, sind geeignete Ausgangsstoffe. Ebenfalls geeignet sind die jeweils aus Mono- und Diacetaten bestehenden Ester-Gemische, die sich von aromatischen oder alicyclischen Diolen ableiten, wie z. B. 2-henyl-ethan-1.2-diol oder Cyclohexandiol-1.2. Bei der Reaktion wird als Alkanol der Formel IV vorzugsweise Methanol und Ethanol, insbesondere aber Methanol eingesetzt, da die hieraus gebildeten Ester der Formel V besonders flüchtig und daher besonders leicht von den gebildeten wenig flüchtigen vicinalen Diolen abtrennbar sind. Geht man von Diolestern mit höheren Carbonsäuren aus und/oder setzt man die Diolester mit höheren Alkanolen um, ist es zur Erzielung eines möglichst hohen Umsatzes zweckmäßig, die Umsetzung bei höheren Reaktionstemperaturen, vermindertem Druck und höherem Alkanolüberschuß durchzuführen. Im allgemeinen setzt man einen auf die Estergruppen des Diolesters bezogenen Überschuß von Alkanol der Formel IV ein ; bewährt hat sich ein Verhältnis von 1.1 bis 5 Mol Alkanol pro Mol Estergruppe.

Bevorzugte Katalysatoren sind starke anorganische oder organische Basen, z. B. Natrium- oder Kaliumhydroxid oder Natrium-Methylat, die dem Reaktionsgemisch in einer Konzentration von 0,5-5, vorzugsweise 1-3 Gew.-% bezogen auf den Diolester zugesetzt werden. Auch starke Säuren wie Schwefelsäure oder Perchlorsäure können verwendet werden ; ihre katalytische Aktivität ist jedoch geringer als die der starken Basen.

Die Umsetzung des Diolesters mit dem niederen Alkanol erfolgt in der Schmelze unter Normaldruck oder vermindertem Druck bei 20 bis 100 °C, vorzugsweise bei 40 bis 80 °C innerhalb von etwa 10 bis 60 Minuten, während überschüssiges Alkanol zusammen mit dem gebildeten flüchtigen Ester abdestilliert wird. Man erhält in praktisch vollständigem Umsatz des Diolesters als Destillationsrückstand vicinales Diol in hoher Reinheit, da kaum Nebenreaktionen stattfinden. Im Vergleich zu den bekannten Verfahren des Standes der Technik sind bei dem erfindungsgemäßen Verfahren lediglich milde Reaktionsbedingungen und nur ein geringer Chemikalieneinsatz erforderlich. Die erhaltenen vicinalen Diole sind meist ohne weitere Reinigung in kosmetischen Präparationen als hautfreundliche Zusätze, als antimikrobielle Substanzen, als Gleitmittel für Kunststoffe oder als Schmiermittel für Maschinenbauteile verwendbar. Bei Bedarf können die Diole durch übliche Reinigungsverfahren, z. B. durch Destillation oder Umkristallisieren leicht weiter gereinigt werden.

## Beispiele

Das Beispiel 1 beschreibt die im Prinzip aus der Literatur bekannte Herstellung von Diolestern, die als Ausgangsprodukt für die Herstellung von vicinalen Diolen nach dem erfindungsgemäßen Verfahren dienen.

### Beispiel 1

940 g $C_{12/14}$-Alkan-1.2-epoxid (4,3 Mol) ließ man mit 260 g Essigsäure (4,3 Mol) in Gegenwart von 10 g

Natriumacetat in einem Rührgefäß bei 130 bis 150 °C 4 Stunden lang reagieren. Dann destillierte man nicht umgesetzte Essigsäure und flüchtige Nebenbestandteile unter vermindertem Druck ab. Der Destillationsrückstand (1 180 g) bestand zu 95 Gew.-% ( = 4,08 Mol) aus $C_{12/14}$-Diolester, vorwiegend dem Monoacetat.

Die folgenden Beispiele sind Beispiele für das erfindungsgemäße Verfahren. Bei den eingesetzten Diolestern handelt es sich, wenn nicht anders angegeben, um vorwiegend aus Monoestern mit geringen Mengen Diester und Diol bestehende Gemische.

Beispiel 2

Der Diolester des Beispiels 1 (mit 4.08 Mol Diolester) wurde bei 70 °C unter Rühren mit 392 g (12,3 Mol) Methanol und 30 g Natriummethylat versetzt. Innerhalb von 30 Minuten wurden das gebildete Methylacetat und überschüssiges Methanol unter Normaldruck abdestilliert. Der Destillationsrückstand (1 040 g) enthielt 83,4 Gew.-% ( = 4,08 Mol) $C_{12}/_{14}$-Alkan-1.2-diol. Die Bestimmung des Diol-Gehaltes erfolgte durch Titration (nach Vic. Mehlenbacher in J. Michell, Org. Anal., Vol. 1, S. 44, N. Y. 1953) und durch Ermittlung von Kennzahlen.

Beispiel 3

100 g eines vorwiegend aus dem Monoacetat des n-$C_{12/14}$-Alkan-1.2-diols bestehenden Diolesters (mit 0,36 Mol $C_{12/14}$-Diolester) wurden mit 23 g (0,72 Mol) Methanol, in dem 2 g Kaliumhydroxid gelöst waren, versetzt. Bei 50 °C wurden im Wasserstrahlvakuum innerhalb von 15 Minuten unter Rühren überschlüssiger Methanol und gebildetes Methylacetat abdestilliert. Der Destillationsrückstand (88,7 g) enthielt 83,7 Gew.-% ( = 0,35 Mol) n-$C_{12/14}$-Alkan-1.2-diol.

Beispiel 4

100 g eines vorwiegend aus dem Monoacetat des vicinalen n-$C_{11/14}$-Alkan-diols mit innenständiger, willkürlich verteilter Diolgruppe bestehenden Produktes mit 0,34 Mol n-$C_{11/14}$-Diolester wurden mit 53 g (1,65 Mol) Methanol und 3 g Kaliumhydroxid versetzt. Bei 50 °C wurden innerhalb von 20 Minuten unter vermindertem Druck die flüchtigen Bestandteile abdestilliert. Der Destillationsrückstand (89,3 g) enthielt 77,9 Gew.-% ( = 0,32 Mol) vic. $C_{11/14}$-Alkan-diol (innenständig, willkürlich verteilt.).

Beispiel 5

100 g eines destillierten, vorwiegend aus dem Monoacetat des n-Hexan-1.2-diol bestehenden Diolesters (0,62 Mol) wurden mit 100 g (3,1 Mol) Methanol und 2 g Natriummethylat versetzt. Bei 50 °C wurden unter vermindertem Druck die flüchtigen Bestandteile abdestilliert. Der Destillationsrückstand enthielt 94,5 Gew.-% ( = 0,59 Mol) n-Hexan-1.2-diol.

Beispiel 6

10 g (0,27 Mol) n-Eicosan-1.2-diolacetat wurden mit 43 g (1,34 Mol) Methanol und 2 g Natriummethyl versetzt. Der nach Abdestillieren bei 80 °C verbliebene Destillationsrückstand von 91,5 g enthielt 85,1 Gew.-% (n 0,26 Mol) n-Eicosan-1.2-diol.

Beispiel 7

100 g (0,28 Mol) n-Tetradecan-1.2-diolformiat wurden mit 17 g (0,53 Mol) Methanol in Gegenwart von 2 g Natriummethylat umgesetzt. Nach Abdestillieren bei 60 °C unter vermindertem Druck erhielt man einen Rückstand von 92,2 g mit 69 Gew.-% (0,286 Mol) n-Tetradecan-1.2-diol.

Beispiel 8

100 g (0,36 Mol) n-$C_{12/14}$-Alkan-1.2-diol-monoacetat wurden mit 105 g (1,75 Mol) n-Propylalkohol und 2 g Natriummethylat umgesetzt. Nach Abdestillieren bei 70 °C unter vermindertem Druck verblieben 88,8 g mit 82,9 Gew.-% ( = 0,356 Mol) n-$C_{12/14}$-Alkan-1.2-diol.

Beispiel 9

100 g des Diolesters aus Beispiel 8 wurden mit 133,5 g (1,8 Mol) n-Butylalkohol in Gegenwart von 2 g Natriummethylat umgesetzt. Nacht Abdestillieren bei 70 °C unter vermindertem Druck verblieben 88,8 g mit 83,8 Gew.-% ( = 0,36 Mol) n-$C_{12/14}$-Alkan-1.2-diol.

Beispiel 10

Es wurde verfahren wie in Beispiel 9 jedoch mit 53,4 (0,72 Mol) n-Butylalkohol. Man erhielt 91,9 g Rückstand mit 70,3 Gew.-% ( = 0,30 Mol) Diol.

Beispiel 11

46,3 g eines vorwiegend aus dem Gemisch eines Mono- und Diacetats des 2 Phenyl-ethan-1.2-diol bestehenden Produkts (0,23 Mol Phenylglykolester) wurden mit 28,8 g Methanol (0,9 Mol) in Gegenwart von 0,9 g Kaliumhydroxid umgesetzt. Es wurde bei 70 °C unter vermindertem Druck abdestilliert. Dabei verblieben 35,0 g Produkt, das 0,232 Mol Phenylglykol enthielt.

Beispiel 12

36,7 g eines vorwiegend aus dem Mono- und Diacetat des Cyclohexan-diol-1.2 bestehenden Produktes (0,217 Mol Cyclohexandiolester) wurden mit 48 g Methanol (1,5 Mol) in Gegenwart von 0,6 g Kaliumhydroxid umgesetzt. Es wurde bei ca. 80 °C unter vermindertem Druck abdestilliert. Dabei verblieben 26,6 g Produkt mit 91,3 Gew.-% (0,209 Mol) Cyclohexandiol-1.2.

Beispiel 13

57,7 g eines vorwiegend an dem Monoacetat des n-Tetradecan-1.2-diol bestehenden Esters (0,2 Mol) wurden mit 32 g (1 Mol) Methanol und 2 g konz. Schwefelsäure versetzt. Nach Abdestillieren bei 60 °C unter vermindertem Druck verblieben 53,6 g mit 63,6 Gew.% ( = 0,198 Mol) n-Tetradecan-1.2-diol.

**Ansprüche**

1. Verfahren zur Herstellung von Diolen aus Diolestern von niederen Carbonsäuren durch Umesterung mit niederen Alkoholen in Gegenwart von sauren oder basischen Katalysatoren, wobei die sich bildenden, leicht flüchtigen Ester aus niederen Carbonsäuren abgetrennt werden, dadurch gekennzeichnet, daß man Ester aus vicinalen Diolen mit wenigstens 6 Kohlenstoffatomen und niederen Carbonsäuren mit niederen Alkoholen umsetzt.

2. Verfahren zur Herstellung vicinaler Diole der Formel

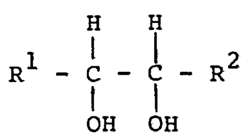

(I)

in der $R^1$ und $R^2$ Wasserstoff oder einen aliphatischen oder aromatischen Kohlenwasserstoff-Rest, der gegebenenfalls auch substituiert oder durch Heteroatome unterbrochen sein kann, mit 1-26 Kohlenstoffatomen bedeuten, mit der Maßgabe, daß $R^1$ und $R^2$ zusammen 4-28 Kohlenstoffatome aufweisen, aus Estern vicinaler Diole nach Anspruch 1, dadurch gekennzeichnet, daß man Ester der Formel II

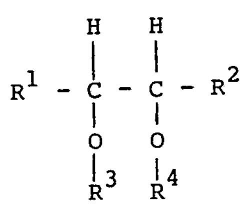

(II)

in der $R^1$ und $R^2$ die oben angegebenen Bedeutungen haben und von den Resten $R^3$ und $R^4$ wenigstens einer einen Acylrest der Formel III

$$—OC—R^5 \qquad (III)$$

darstellt, in der $R^5$ Wasserstoff oder einen aliphatischen Rest mit 1-4 Kohlenstoffatomen bedeutet, während der andere Rest entweder Wasserstoff oder einen Acylrest der Formel III darstellt, mit einem Alkanol der Formel IV

$$R^6—OH \qquad (IV)$$

in der $R^6$ einen aliphatischen Rest mit 1-4 Kohlenstoffatomen bedeutet, in Gegenwart von stark basischen oder sauren Katalysatoren unter Bildung von vicinalen Diolen der Formel I und Estern der Formel V

$$R^6—O—OC—R^5 \qquad (V)$$

in der $R^5$ und $R^6$ die für die Formeln III und IV genannten Bedeutungen haben, umsetzt und den Ester der Formel V von den Diolen der Formel I abdestilliert.

3. Verfahren nach Anspruch 1 und 2, dadurch gekennzeichnet, das man Diolester der Formel II einsetzt, in deren Acylrest der Formel III $R^5$ Wasserstoff oder den Methylrest bedeutet.

4. Verfahren nach Anspruch 1 bis 3, dadurch gekennzeichnet, daß man als Alkanol der Formel IV Methanol oder Ethanol, vorzugsweise Methanol einsetzt.

5. Verfahren nach Anspruch 1 bis 4 dadurch gekennzeichnet, daß man pro Mol Estergruppe 1,1 bis 5 Mol Alkanol der Formel IV einsetzt.

6. Verfahren nach Anspruch 1 bis 5, dadurch gekennzeichnet, daß man als Katalysator 0,5-5, vorzugsweise 1-3 Gew.-%, bezogen auf den Diolester, basische Verbindungen aus der Gruppe Natriumhydroxid, Kaliumhydroxid und Natriummethylat einsetzt.

7. Verfahren nach Anspruch 1 bis 6, dadurch gekennzeichnet, daß man die Umsetzung in flüssiger Phase bei 20 bis 100 °C, vorzugsweise bei 40 bis 80 °C durchführt.

8. Verfahren nach Anspruch 1 bis 7, dadurch gekennzeichnet, daß man die Umsetzung des Diolesters und Abtrennung des gebildeten Esters der Formel V unter Normaldruck durchführt.

9. Verfahren nach Anspruch 1 bis 7, dadurch gekennzeichnet, daß man die Umsetzung des Diolesters und Abtrennung des gebildeten Esters der Formel V unter vermindertem Druck durchführt.

10. Verfahren nach Anspruch 8 und 9, dadurch gekennzeichnet, daß man die Umsetzung des Diolesters und Abtrennung des gebildeten Esters der Formel V innerhalb von 10 bis 60 Minuten durchführt.

**Claims**

1. A process for the production of diols from diol esters of lower carboxylic acids by transesterification with lower alcohols in the presence of acid or basic catalysts, the readily volatile esters of lower carboxylic acids formed being separated off, characterized in that esters of vicinal diols containing at least 6 carbon atoms and lower carboxylic acids are reacted with lower alcohols.

2. A process for the production of vicinal diols corresponding to the following formula

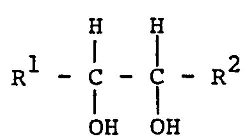

(I)

in which $R^1$ and $R^2$ represent hydrogen or a $C_{1-26}$ aliphatic or aromatic hydrocarbon radical which may even be substituted or interrupted by hetero atoms, with the proviso that $R^1$ and $R^2$ together contain from 4 to 28 carbon atoms, from esters of vicinal diols as claimed in Claim 1, characterized in that esters corresponding to the following formula

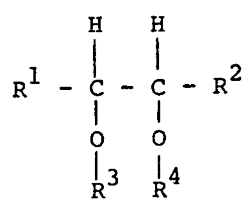

(II)

in which $R^1$ and $R^2$ are as defined above and at least one of the radicals $R^3$ and $R^4$ is an acyl radical corresponding to the following formula

$$—OC—R^5 \qquad (III)$$

where $R^5$ is hydrogen or an aliphatic radical containing from 1 to 4 carbon atoms, whilst the other radical is either hydrogen or an acyl radical of formula III, are reacted with an alkanol corresponding to the following formula

$$R^6—OH \qquad (IV)$$

in which $R^6$ is an aliphatic radical containing from 1 to 4 carbon atoms, in the presence of strongly basic or acid catalysts to form vicinal diols corresponding to formula I and esters corresponding to the following formula

$$R^6—O—OC—R^5 \qquad (V)$$

in which $R^5$ and $R^6$ have the same meanings as in formulae III and IV, and the ester of formula V is distilled of from the diols of formula I.

3. A process as claimed in Claims 1 and 2, characterized in that diol esters of formula II, in whose acyl radical of formula III $R^5$ is hydrogen or the methyl radical, are used.

4. A process as claimed in Claims 1 to 3, characterized in that methanol or ethanol, preferably methanol, is used as the alkanol of formula IV.

5. A process as claimed in Claims 1 to 4, characterized in that from 1.1 to 5 moles of the alkanol of formula IV are used per mole of ester group.

6. A process as claimed in Claims 1 to 5, characterized in that basic compounds from the group comprising sodium hydroxide, potassium hydroxide and sodium methylate are used as catalyst in quantities of from 0.5 to 5 % by weight and preferably in quantities of from 1 to 3 % by weight, based on the diol ester.

7. A process as claimed in Claims 1 to 6, characterized in that the reaction is carried out in the liquid phase at temperatures of from 20 to 100 °C and preferably at temperatures in the range from 40 to 80 °C.

8. A process as claimed in Claims 1 to 7, characterized in that the reaction of the diol ester and separation of the ester of formula V formed are carried out under normal pressure.

9. A process as claimed in Claims 1 to 7, characterized in that the reaction of the diol ester and separation of the ester of formula V formed are carried out under reduced pressure.

10. A process as claimed in Claims 8 and 9, characterized in that the reaction of the diol ester and separation of the ester of formula V formed are carried out over a period of from 10 to 60 minutes.

**Revendications**

1. Procédé de préparation de diols à partir d'esters de diols d'acides carboxyliques inférieurs par transestérification à l'aide d'alcools inférieurs en présence de catalyseurs acides ou basiques dans lequel on sépare les esters volatils d'acides carboxyliques inférieurs formés, caractérisé en ce que l'on fait réagir des esters de diols vicinaux à au moins 6 atomes de carbone et d'acides carboxyliques inférieurs avec des alcools inférieurs.

2. Procédé de préparation de diols vicinaux de formule

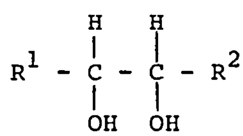

(I)

dans laquelle $R^1$ et $R^2$ représentent l'hydrogène ou un reste hydrocarboné aliphatique ou aromatique qui peut éventuellement être substitué ou interrompu par des hétéroatomes et qui contient de 1 à 26 atomes de carbone, étant spécifié que $R^1$ et $R^2$ ont ensemble 4 à 28 atomes de carbone, à partir d'esters de diols vicinaux selon la revendication 1, caractérisé en ce que l'on fait réagir des esters de formule II

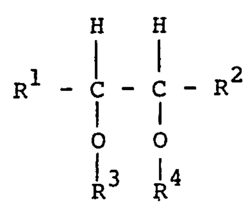

(II)

dans laquelle $R^1$ et $R^2$ ont les significations indiquées ci-dessus et l'un au moins des symboles $R^3$ et $R^4$ représente un radical acyle de formule III

$$—OC—R^5 \qquad (III)$$

dans laquelle $R^5$ représente l'hydrogène ou un reste aliphatique en C1-C4, l'autre représentant l'hydrogène ou un radical acyle de formule III avec un alcanol de formule IV

$$R^6—OH \qquad (IV)$$

dans laquelle $R^6$ représente un reste aliphatique en C1-C4, en présence de catalyseurs fortement basiques ou acides, avec formation de diols vicinaux de formule I et d'esters de formule V

$$R^6—O—OC—R^5 \qquad (V)$$

dans laquelle $R^5$ et $R^6$ ont les significations indiquées en référence aux formules III et IV, et on sépare l'ester de formule V des diols de formule I par distillation.

3. Procédé selon les revendications 1 et 2, caractérisé en ce que l'on utilise des esters de diols de formule II ayant un radical acyle de formule III dans lequel $R^5$ représente l'hydrogène ou le groupe méthyle.

4. Procédé selon les revendications 1 à 3 caractérisé en ce que l'on utilise en tant qu'alcanol de formule IV l'éthanol ou le méthanol, de préférence le méthanol.

5. Procédé selon les revendications 1 à 4 caractérisé en ce que l'on utilise de 1,1 à 5 moles de l'alcanol de formule IV par mole de groupe ester.

6. Procédé selon les revendications 1 à 5 caractérisé en ce que l'on utilise en tant que catalyseur, en proportion de 0,5 à 5 %, de préférence de 1 à 3 % en poids, par rapport à l'ester de diol, un composé basique pris dans le groupe formé par l'hydroxyde de sodium, l'hydroxyde de potassium et le méthylate de sodium.

7. Procédé selon les revendications 1 à 6, caractérisé en ce que l'on effectue la réaction en phase liquide à une température de 20 à 100 °C de préférence de 40 à 80 °C.

8. Procédé selon les revendications 1 à 7, caractérisé en ce que l'on effectue la réaction de l'ester de diol et la séparation de l'ester formé de formule V à pression normale.

9. Procédé selon les revendications 1 à 7, caractérisé en ce que l'on procède à la réaction de l'ester de diol et à la séparation de l'ester formé de formule V sous vide.

10. Procédé selon les revendications 8 et 9, caractérisé en ce que l'on procède à la réaction de l'ester de diol et à la séparation de l'ester formé de formule V en une durée de 10 à 60 minutes.